# FASCICULE DE BREVET EUROPEEN

(11) **EP 2 265 364 B1**
(45) Date de publication et mention de la délivrance du brevet: **13.07.2016**
(21) Numéro de dépôt: 09745925.9
(22) Date de dépôt: 14.04.2009
(51) Int. Cl.: B01J 13/04

(54) **NOUVEAU PROCEDE DE FABRICATION DE NANOCAPSULES, EN L'ABSENCE DE SOLVANT ORGANIQUE, ET NANOCAPSULES AINSI OBTENUES**
NEUARTIGES VERFAHREN ZUR HERSTELLUNG VON NANOKAPSELN OHNE VERWENDUNG EINES ORGANISCHEN LÖSUNGSMITTELS SOWIE MIT DIESEM VERFAHREN HERGESTELLTE NANOKAPSELN
NOVEL METHOD FOR PRODUCING NANOCAPSULES IN THE ABSENCE OF AN ORGANIC SOLVENT, AND NANOCAPSULES PRODUCED THEREBY

(30) Priorité: 18.04.2008 FR 0852648
(43) Date de publication de la demande: 29.12.2010
(73) Titulaire: Université Claude Bernard Lyon I, 69622 Villeurbanne Cedex (FR)
(72) Inventeur: PIROT, Fabrice, F-69006 Lyon (FR); FALSON, Françoise, F-69890 La Tour De Salvagny (FR)
(74) Mandataire: Sarlin, Laure V.
(86) Numéro de dépôt international: PCT/FR2009/050680
(87) Numéro de publication internationale: WO 2009/138606

(56) Documents cités:
- FR-A- 2 766 368
- LBOUTOUNNE H ET AL.: "Sustained ex vivo skin antiseptic activity of chlorhexidine in poly(epsilon-caprolactone) nanocapsule encapsulated form and as a digluconate" JOURNAL OF CONTROLLED RELEASE, ELSEVIER, AMSTERDAM, NL, vol. 82, no. 2-3, 21 août 2002 (2002-08-21), pages 319-334, XP004374931 ISSN: 0168-3659
- LBOUTOUNNE H ET AL.: "Characterization of Transport of Chlorhexidine-Loaded Nanocapsules through Hairless and Wistar Rat Skin" SKIN PHARMACOLOGY AND PHYSIOLOGY: JOURNAL OF PHARMACOLOGICAL ANDBIOPHYSICAL RESEARCH, S. KARGER AG, BASEL, CH, vol. 17, 1 janvier 2004 (2004-01-01), pages 176-182, XP008099005 ISSN: 1660-5527
- DANG THI TUYET NHUNG ET AL.: "Sustained antibacterial effect of a hand rub gel incorporating chlorhexdine-loaded nanocapsules (Nanochlorex)" INTERNATIONAL JOURNAL OF PHARMACEUTICS, vol. 334, no. 1-2, 13 octobre 2006 (2006-10-13), pages 166-172, XP002505904
- JAMEELA S.R. ET AL.: "Protein release from poly(epsilon-caprolactone) microspheres prepared by melt encapsulation and solvent evaporation techniques: a comparative study" JOURNAL OF BIOMATERIALS SCIENCE, POLYMER EDITION, vol. 8, no. 6, 1997, pages 457-466, XP002505905
- QUAGLIA ET AL: "Nanoscopic core-shell drug carriers made of amphiphilic triblock and star-diblock copolymers" INTERNATIONAL JOURNAL OF PHARMACEUTICS, ELSEVIER BV, NL, vol. 324, no. 1, 31 octobre 2006 (2006-10-31), pages 56-66, XP005707831 ISSN: 0378-5173
- E. MATHIOWITZ ET AL.: "Polyanhydride microspheres as drug carriers I. Hot-melt microencapsulation" JOURNAL OF CONTROLLED RELEASE, vol. 5, no. 1, 8 novembre 2002 (2002-11-08), pages 13-22, XP002505906

## Description

La présente invention concerne le domaine technique des nanocapsules, en particulier des nanocapsules utiles comme agents transporteurs de principe actif.

Plus précisément, l'invention a pour objet un procédé de fabrication de nanocapsules, ainsi que les nanocapsules susceptibles d'être obtenues par un tel procédé.

Les nanoparticules appartiennent à une famille de vecteurs colloïdaux utilisables, par exemple, pour l'administration de principes actifs, telles que des molécules thérapeutiques chez l'Homme ou l'animal. De tels vecteurs colloïdaux préservent ces principes actifs et peuvent permettre leur libération contrôlée et/ou prolongée au niveau de leur site d'action. Les nanocapsules et les nanosphères constituent deux groupes distincts de nanoparticules. Les nanocapsules sont formées d'un coeur aqueux ou huileux recouvert par une membrane polymérique ; les nanosphères sont formées d'une matrice polymérique. Les polymères entrant dans la composition des nanoparticules sont caractérisés par une forte hydrophobie (e.g., polyesters).

FR 2 766 368 A décrit un procédé de préparation d'une suspension aqueuse de nanocapsules comprenant un coeur huileux S3 entouré par une enveloppe polymérique PE dans lequel on mélange a) une première phase comportant un polymère PE, une huile S3, un principe actif PA ,un tensioactif stabilisant et b) une deuxième phase comportant un non-solvant S2 du polymère PE, un tensioactif stabilisant de manière à obtenir la formation des nanocapsules en suspension aqueuse.

La production de suspension aqueuse de nanocapsules polymériques est réalisée, le plus souvent, selon deux techniques :
- par dépôt interfacial de polymère (H. Fessi, F. Puisieux, J. P. Devissaguet, N. Ammoury, and S. Benita. Nanocapsule formation by interfacial polymer deposition following solvent displacement, International Journal of Pharmaceutics 5 5: R1-R4 (1989) ;
- par émulsion-diffusion (D. Moinard-Checot, Y. Chevalier, S. Briancon, L. Beney, and H. Fessi. Mechanism of nanocapsules formation by the emulsion-diffusion process. Journal cf Colloid and Interface Science In Press, Corrected Proof: 77).

Ces deux techniques requièrent l'emploi de solvants organiques pour la dissolution des polymères hydrophobes, constitutifs de la membrane polymérique des nanocapsules.

La technique d'obtention de suspension aqueuse de nanoparticules polymériques par dépôt interfacial de polymère, méthode la plus commune pour la production de nanocapsules, est basée sur le dépôt de polymères préformés à l'interface entre (i) un solvant organique mélangé à une huile et (ii) une solution aqueuse. Dans ce procédé, on prépare une solution contenant, e.g., un principe actif dans un solvant organique soluble ou très soluble dans l'eau tel que l'acétone (avec ou sans surfactant lipophile). Une huile miscible dans le solvant organique mais non miscible dans l'eau est ajoutée à la solution organique précédente. La solution organique est alors dispersée, par agitation mécanique, dans la phase polaire contenant le plus souvent un surfactant hydrophile (e.g., poloxamer, polysorbate 80). Le solvant organique diffuse dans la phase polaire avec pour conséquence une agrégation du polymère autour des gouttelettes lipidiques responsable de la formation des nanocapsules. Le solvant organique peut être dispersé dans la phase polaire par filtration tangentielle sur membrane suivie d'une agitation mécanique (I. Limayem Blouza, C. Charcosset, S. Sfar and H. Fessi. Preparation and characterization of spironolacton-loaded nanocapsules for paediatric use International Journal of Pharmaceutics 325:124-131(2006). Le solvant organique est éliminé, soit par dialyse, soit, le plus souvent, par évaporation sous pression réduite.

La technique par émulsion-diffusion (D. Moinard-Checot, Y. Chevalier, S. Briancon, L. Beney, and H. Fessi. Mechanism of nanocapsules formation by the emulsion-diffusion process. Journal of Colloid and Interface Science In Press, Corrected Proof: 77), quant à elle, consiste à former une émulsion où la phase huileuse contient un polymère biodégradable et un solvant partiellement miscible à l'eau (e.g., acétate d'éthyle, solubilité : 8,3 g/100 ml à 20°C) et ou la phase polaire contenant un émulsifiant est saturée en acétate d'éthyle. L'addition d'eau à cette émulsion provoque la diffusion du solvant des gouttelettes d'huile vers la phase externe, ce qui entraîne la précipitation du polymère autour des gouttelettes d'huile, et donc la formation de nanocapsules. Le solvant organique est éliminé soit par dialyse, soit le plus souvent par évaporation sous pression réduite.

Par conséquent, les modes actuels de production des nanocapsules comportent tous une étape préliminaire de dissolution des polymères hydrophobes dans un mélange d'un ou plusieurs solvants organiques volatils (e.g., acétone, acétate d'éthyle). La formation « spontanée » des nanocapsules est assurée par la dispersion des solutions organiques de polymères hydrophobes dans un mélange polaire (e.g., solutions aqueuses le plus souvent). La dernière étape de production impose une évaporation sous vide ou une dialyse des solvants organiques. Le contrôle de l'élimination de ces solvants et la détection de leurs traces éventuelles dans la préparation finale sont autant de procédures longues et coûteuses qui requièrent, à l'échelle industrielle, la conception et la mise en place d'infrastructures complexes.

Dans ce contexte, la présente invention se propose de fournir un nouveau procédé de préparation de nanocapsules qui soit plus facile à mettre en oeuvre, et qui soit adapté à une échelle industrielle.

Par ailleurs, l'un des objectifs essentiels de la présente invention est de fournir un procédé de préparation de nanocapsules qui soient exemptes de produits toxiques et stables en suspension colloïdale et qui permette une bonne protection du principe actif encapsulé, de même qu'une libération prolongée et/ou contrôlée de celui-ci *in vivo.*

Un autre objectif essentiel de la présente invention est de fournir un procédé de préparation de nanocapsules du type de celui sus-visé et qui soit en outre aisé et rentable à mettre en oeuvre à l'échelle industrielle car ne nécessitant pas d'étapes de purification lourdes et coûteuses.

Un autre objectif de l'invention est de fournir un procédé de préparation de nanocapsules permettant d'obtenir de manière fiable et reproductible des nanocapsules de taille inférieure à 1 µm.

Le procédé selon l'invention ne met en oeuvre aucun solvant organique, ce qui permet de l'exonérer des étapes longues et coûteuses de l'élimination du solvant organique, incontournables dans les techniques de l'art antérieur.

Dans ce contexte, l'invention concerne un procédé de préparation d'une suspension aqueuse de nanocapsules comprenant un coeur huileux entouré par une enveloppe polymérique dans lequel on mélange :
a) une première phase, nommée phase huileuse comportant :
   - un polymère hydrophobe,
   - une huile, ou un mélange d'huiles,
   - au moins un principe actif,
   - et un agent tensio-actif **TA₁,**
   cette phase huileuse étant portée à une température **T₁** supérieure à la température de fusion du polymère hydrophobe, à cette température **T₁,** le polymère hydrophobe étant miscible au mélange de l'agent tensio-actif **TA₁** et de l'huile ou du mélange d'huiles, et le principe actif étant miscible, soluble ou solubilisé, dans le mélange de l'agent tensio-actif **TA₁** et de l'huile ou du mélange d'huiles;
b) une deuxième phase, nommée phase polaire, comportant un polymère hydrophile sous la forme d'un hydrogel dans une solution aqueuse contenant un agent tensio-actif **TA₂,**
de manière à obtenir la formation des nanocapsules en suspension aqueuse.

L'invention a également pour objet des nanocapsules susceptibles d'être obtenues selon le procédé de l'invention, notamment des nanocapsules comprenant un coeur huileux dans lequel un principe actif est dispersé de façon homogène et une enveloppe polymérique formée d'un polymère hydrophobe et d'un polymère hydrophile, susceptibles d'être obtenues selon le procédé de l'invention, ainsi que des suspensions aqueuses de telles nanocapsules.

Selon un autre de ses aspects, l'invention concerne les compositions pharmaceutiques, cosmétiques ou alimentaires, comprenant des nanocapsules selon l'invention ou une suspension aqueuse de nanocapsules selon l'invention, en combinaison avec au moins un excipient pharmaceutiquement ou physiologiquement acceptable.

Certaines définitions de termes utilisés dans le cadre de la description de l'invention sont données ci-après.

Les notions de solubilité, miscibilité et solubilisation sont bien connues de l'homme du métier. On pourra notamment se référer à Physical Pharmacy (4ème édition, Alfred Martin (ed.), Lea & Febiger Philadelphia, London) chapitre 10, pp212-250, chapitre 15, pp393-422. Sauf indications contraires, dans le cadre de l'invention, la solubilité, miscibilité ou solubilité est obtenue à température ambiante, notamment à 20°C.

En particulier, dans le cadre de l'invention, par « miscible », on entend complètement miscible. Deux composés liquides seront considérés comme complètement miscibles lorsqu'ils se mélangent en toute proportion. Par conséquent, le terme miscibilité se réfère à la solubilité mutuelle des composés dans les systèmes liquides.

Notamment, au sens de l'invention, un composé solide sera considéré comme soluble dans un liquide ou mélange de liquides lorsque ce composé se disperse de manière homogène à l'état moléculaire sous l'effet d'interactions solide/liquide spontanées. Un principe actif est considéré comme soluble dans un liquide ou un mélange de liquides lorsque 1 g de principe actif est dissout dans 10 à 30 ml de liquide ou mélange de liquides (Pharmacopée US).

Concernant la solubilisation, un composé solide ou liquide (minéral ou organique) sera considéré comme solubilisé dans un liquide ou mélange de liquides, notamment lorsque une association de colloïdes formant des micelles augmente la solubilité du composé initialement insoluble dans le milieu de dispersion.

Par polymère « hydrophile », on entend un polymère soluble dans l'eau. Par polymère soluble en solution aqueuse, on entend un polymère qui, introduit dans de l'eau à 20 °C, à une concentration en poids égale à 1%, permet l'obtention d'une solution qui présente une valeur de transmittance maximale de la lumière, à une longueur d'onde à laquelle le polymère d'absorbe pas, à travers un échantillon de 1 cm d'épaisseur, d'au moins 70%, de préférence d'au moins 80%.

Par polymère « hydrophobe », on entend un polymère insoluble dans l'eau.

Par « huile », on entend un corps gras, lipophile, liquide à la température ambiante (20°C) qui est non-miscible à l'eau ou faiblement miscible à l'eau. Par huile « hydrodispersible », on entend une huile qui se disperse dans l'eau à l'état moléculaire, colloïdal ou micrométrique.

Le HLB (de l'anglais « hydrophilic lipophilic balance ») sera déterminé par la méthode de Griffin. (Griffin WC: Classification of Surface-Active Agents by 'HLB,' Journal of the Society of Cosmetic Chemists 1 (1949): 311. Griffin WC: Calculation of HLB Values of Non-Ionic Surfactants, Journal of the Society of Cosmetic Chemists 5 (1954): 259).

Le diamètre des nanocapsules, qui correspond à la plus grande dimension des nanocapsules, sera déterminé par spectroscopie de corrélation de photons.

Par « hydrogel », on entend un mélange homogène gélatineux formant une seule phase contenant de l'eau, et de préférence comportant au moins 0,1 à 5% en masse, de préférence 0,15 à 2% en masse d'eau.

Le procédé selon l'invention permet d'obtenir une suspension aqueuse de nanocapsules, par un procédé d'émulsion gélification, qui ne fait intervenir aucun solvant organique.

De ce fait, le nouveau procédé de préparation d'une suspension aqueuse de nanocapsules selon l'invention se distingue des autres techniques décrites précédemment :
- par l'absence de solvants organiques utilisés pour dissoudre les polymères hydrophobes avant dispersion du mélange organique dans la phase polaire ;
- par l'absence de procédure d'évaporation ou de dialyse de solvants organiques après formation des nanocapsules ;
- par la production d'une suspension de nanocapsules compatible avec une administration invasive ou non invasive.

Le polymère hydrophile présent au sein de l'enveloppe polymérique. permet de réduire la tension interfaciale existant entre le polymère hydrophobe et l'eau, et augmente ainsi la stabilité de la suspension obtenue. Il est ainsi possible d'obtenir spontanément la formation de nanocapsules lors du mélange des deux phases et ce, sans l'usage de solvant organique volatile, comme c'était le cas dans les techniques antérieures.

Dans le cadre de l'invention deux phases, nommées phase huileuse et phase polaire, sont mélangées pour conduire à la formation spontanée de nanocapsules. Les pourcentages donnés ci-dessous correspondent :
- en ce qui concerne la phase huileuse, au pourcentage en masse de chaque composant sur la masse totale de la phase huileuse,
- en ce qui concerne la phase polaire, au pourcentage en masse de chaque composant sur la masse totale de la phase polaire.

La phase huileuse comprend un polymère hydrophobe, une huile, ou un mélange d'huiles, au moins un principe actif, et un agent tensio-actif **TA₁.** Cette phase huileuse est homogène.

L'huile ou les huiles utilisées sont, par nature hydrophobes, et peuvent dans certains cas être hydrodispersibles. Cette huile ou mélange d'huiles est destinée à former le coeur des nanocapsules.

En particulier, l'huile ou le mélange d'huiles peut présenter un HLB compris dans la gamme allant de 3 à 6.

A titre d'exemple d'huile pouvant être utilisée dans le cadre de l'invention, on peut citer les triglycérides, notamment à chaîne moyenne, les propylène glycol dicaprylocaprate, les macrogolglycérides oléoyles, lauroyles et linoléoyles.

La phase huileuse comprend, de façon avantageuse, de 5 à 20% en masse, de préférence de 8 à 12% en masse d'huile ou de mélange d'huiles. Bien entendu, ce pourcentage ne concerne que l'huile ou le mélange d'huiles et ne comprend notamment pas le principe actif et/ou l'agent tensio-actif **TA₁,** même quand ces derniers se trouvent également sous une forme huileuse.

La phase huileuse contient un polymère hydrophobe à l'état fondu, la phase huileuse étant maintenue à une température **T**₁ supérieure à la température de fusion du polymère. Le polymère hydrophobe sera choisi de façon à ce que sa température de fusion soit compatible avec la stabilité physicochimique de l'huile, du principe actif et de l'agent tensio-actif **TA₁.** En particulier, le polymère hydrophobe présentera une température de fusion, de préférence inférieure ou égale à 120°C. Notamment, le polymère hydrophobe peut être choisi parmi les polymères vinyliques, les polyesters, les polyamides, les polyuréthanes, les polycarbonates, présentant de préférence une température de fusion inférieure à 120°C, tels que les poly-ε-caprolactones. La phase huileuse peut, notamment, comprendre de 5 à 20% en masse, de préférence de 8 à 12% en masse de polymère hydrophobe.

La phase huileuse contient également un principe actif qui est dispersé, sous une forme miscible, soluble ou solubilisée, dans cette dernière. Le principe actif est miscible, soluble ou solubilisé dans le mélange composé du tensio-actif **TA₁** et de l'huile ou du mélange d'huiles, à la température **T₁.** Selon une variante de réalisation, le principe actif est également miscible, soluble ou solubilisé dans le mélange composé du tensio-actif **TA₁** et de l'huile ou du mélange d'huiles, à température ambiante, notamment à 20°C. Lorsque le principe actif est solubilisé, sa solubilisation est réalisée par l'action du tensio-actif **TA₁,** jouant le rôle d'agent solubilisant.

A titre d'exemple de principe actif, on peut citer la chlorhexidine base, le minoxidil, l'albendazole et le ketoconazole. Par exemple, la phase huileuse comprendra de 0,5 à 5% en masse, de préférence de 1 à 3% en masse de principe actif.

La phase huileuse comprend également un tensio-actif **TA₁,** qui pourra notamment agir en tant qu'agent solubilisant du principe actif. Cet agent tensio-actif **TA₁** peut être du type anionique, cationique, amphotère ou non-ionique. Notamment, l'agent tensio-actif **TA₁** peut se présenter sous la forme d'une huile. L'agent tensio-actif **TA₁** peut présenter un HLB compris dans la gamme allant de 3 à 6. A titre d'exemple d'agent tensio-actif **TA₁,** on peut citer les propylène glycol laurates, les propylène glycol caprylates, les polyglycéryl oléates et les macrogolglycérides caprylocaproyles. Selon un mode de réalisation, la phase huileuse comprend de 55 à 89,5% en masse, de préférence de 73 à 83 % en masse de tensio-actif **TA₁.**

Bien entendu, la phase huileuse pourra contenir un seul ou plusieurs principes actifs et/ou un seul ou plusieurs polymères hydrophobes et/ou un seul ou plusieurs tensio-actifs **TA₁,** répondant aux critères ci-dessus.

La phase huileuse peut, par exemple, être préparée en chauffant le polymère hydrophobe, à une température supérieure à sa température de fusion, puis en ajoutant l'huile ou le mélange d'huiles, puis le principe actif. L'agent tensio-actif **TA₁** peut être introduit à n'importe quel stade. Le mélange peut se faire, dans un tout autre sens ou l'ensemble des composants peuvent tous être mélangés simultanément. Il est également possible de chauffer l'huile ou le mélange d'huiles à la température **T₁,** puis d'ajouter le polymère hydrophobe à l'état liquide, puis les autres composants. La phase huileuse obtenue devra être homogène et si nécessaire sera homogénéisée, par exemple sous agitation mécanique.

La phase polaire, quant à elle, contient en solution aqueuse, un polymère hydrophile sous la forme d'un hydrogel et un agent tensio-actif **TA₂.**

A titre d'exemple, le polymère hydrophile peut être choisi parmi les dérivés cellulosiques synthétiques, de préférence parmi les éthers de cellulose tels que la méthylcellulose, l'éthylcellulose, l'hydroxyéthylcellulose, l'hydroxypropylcellulose, l'hydroxyéthyl-méthylcellulose, l'hydroxypropyl-méthylcellulose, la méthyléthylcellulose et la carboxyméthylcellulose sodique. Selon un mode de réalisation, la phase polaire comprend de 0,1 à 5% en masse, de préférence de 0,15 à 2% en masse de polymère hydrophile.

L'agent tensio-actif **TA₂** peut être du type anionique, cationique, amphotère ou non-ionique. Selon une variante de réalisation, le tensio-actif **TA₂** présente un HLB supérieur ou égal à 15, et est de préférence choisi parmi les tensio-actifs neutres (e.g., polysorbate 20, 60, 80 ; macrogol stéarate ; macrogol cetostearyl ether ; macrogol lauryl ether ; macrogol oleyl ether ; macrogol oléate ; huile de ricin polyoxyl; huile de ricin polyoxyl hydrogénée; source: Eur. Ph.). Selon un mode de réalisation, la phase polaire comprend de 0,1 à 5% en masse, de préférence de 0,2 à 2% en masse de tensio-actif **TA₂.**

Bien entendu, la phase polaire pourra contenir un seul ou plusieurs polymères hydrophiles et/ou un seul ou plusieurs tensio-actif **TA₂,** répondant aux critères ci-dessus.

La phase polaire comprend, le plus souvent, de 90 à 99,8% en masse, de préférence de 96 à 99,65% en masse d'eau ou d'un mélange d'eau avec un ou plusieurs solvants polaires. A titre d'exemple de solvants polaires on peut citer l'éthanol, le 1-propanol et le 2-propanol.

La proportion de phase polaire par rapport à la phase huileuse est variable. On pourra notamment utiliser un rapport en masse de polymère hydrophobe/polymère hydrophile inférieur ou égal à 0,4.

Le mélange entre la phase huileuse et la phase polaire peut être réalisé de diverses manières. Il est possible de verser la phase huileuse dans la phase polaire ou de mélanger les deux phases au moyen d'un circuit mélangeur en forme de Y, chacune des deux phases étant amenée dans un des deux bras du Y. Mais, de façon préférée, le mélange des deux phases est réalisé en ajoutant la phase polaire dans la phase huileuse, sous agitation. La phase huileuse est alors maintenue, lors du mélange, à la température **T₁** souhaitée.

Au moment du mélange, la phase huileuse se trouve à une température **T₁** supérieure à la température de fusion du polymère hydrophobe. On utilisera, notamment, une température de 10 à 30°C supérieure à la température de fusion du polymère hydrophobe. Bien entendu, cette température ne sera pas trop élevée pour éviter toute dégradation des autres composants utilisés dans le procédé selon l'invention. A cette température **T₁**, le mélange composé de l'huile ou du mélange d'huiles et du tensio-actif **TA₁** est miscible au polymère hydrophobe, et le principe actif est également miscible, soluble ou solubilisé, éventuellement grâce à l'agent tensio-actif **TA₁,** dans le mélange composé de l'huile ou du mélange d'huiles et du tensio-actif **TA₁.**

Au moment du mélange ou juste avant le mélange, la phase polaire peut être à température ambiante, notamment à 20°C ou, selon une variante de réalisation, la phase polaire peut être également chauffée. Notamment, la phase polaire portée à une température **T₂** inférieure de 2 à 5°C à la température de fusion du polymère hydrophobe. Il est néanmoins important de conserver, dans les conditions de mélange, le caractère d'hydrogel du polymère hydrophile.

Selon une disposition avantageuse de l'invention, le mélange des deux phases est réalisé sous agitation modérée, de préférence à l'aide de moyens mécaniques fonctionnant à un régime compris dans la gamme allant de 4000 à 16000 tr/min, de préférence dans la gamme allant de 6000 et 8000 tr/min. A titre d'exemple de dispositif d'émulsification convenable pour le procédé selon l'invention, on peut citer un agitateur mécanique à hélice ou un homogénéisateur (ultra-Turrax^{®}).

Le procédé selon l'invention conduit à des nanocapsules de diamètre inférieur à 1000 nanomètres. Ces nanocapsules sont obtenues en suspension aqueuse qui se présente sous la forme d'un mélange homogène gélatineux. Le principe actif peut être encapsulé dans le coeur huileux des nanocapsules, ou bien être adsorbé au sein de l'enveloppe polymérique. Vraisemblablement, le polymère hydrophile forme un colloïde protecteur autour des nanocapsules, assurant une plus grande stabilité de la suspension colloïdale obtenue et une amélioration du processus d'émulsification. La concentration en polymère hydrophile pourra être adaptée, en fonction de l'application visée et, notamment, dans le cas des compositions thérapeutiques, du mode d'administration choisi. La suspension aqueuse gélatineuse de nanocapsules obtenues peut être diluée dans l'eau, sans modification notable de la stabilité de la suspension colloïdale. La charge électrique portée par les nanocapsules sera fonction de la nature et de la concentration du colloïde protecteur venant enrober ou s'enchevêtrer avec le polymère hydrophobe constituant la paroi des nanocapsules. En particulier, les nanocapsules obtenues présentent un diamètre compris dans la gamme allant de 200 à 1000 nm, de préférence dans la gamme allant de 300 à 500 nm. La stabilité en suspension colloïdale de ces nanocapsules est avérée. Elle confère au principe actif qu'elles encapsulent dans leur coeur, ou qui est adsorbé au sein de l'enveloppe polymérique, une protection au stockage, ainsi que lors de leur transport vers leur site d'action. Ces nanoparticules sont donc tout à fait appropriées pour être utilisées comme système colloïdal de vectorisation de principes actifs, notamment de principes actifs pharmaceutiques ou cosmétiques.

Les nanocapsules selon l'invention peuvent être utilisées, notamment dans des compositions pharmaceutiques, cosmétiques ou alimentaires. Dans de telles compositions, les nanocapsules sont, en général, présentes, en association avec au moins un excipient ou véhicule pharmaceutiquement ou physiologiquement acceptable, notamment un excipient qui peut être administré à l'Homme et/ou appliqué sur la peau ou les muqueuses.

Dans le cas où les compositions selon l'invention contiennent un principe actif, choisi parmi les actifs pharmaceutiques ou cosmétiques, à titre d'exemple, on peut citer la chlorhexidine base, le minoxidil, l'albendazole et le ketoconazole. De telles compositions selon l'invention contiennent un milieu ou au moins un excipient pharmaceutiquement ou physiologiquement acceptable. De telles compositions pourront notamment être administrées par voie entérale, parentérale ou topique.

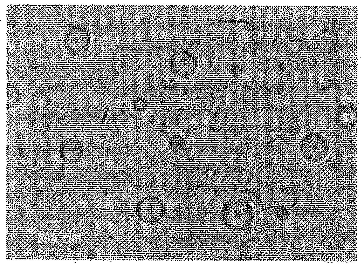

L'exemple ci-après, en référence à la Figure annexée, permet d'illustrer l'invention, mais n'a aucun caractère limitatif.

**EXEMPLE** La composition qualitative et quantitative d'une suspension aqueuse de nanocapsules chargées en chlorhexidine base est donnée ci-dessous :

| | |
|---|---|
| Polycaprolactone (cas #24980-41-4) (PCL) | 375 mg |
| Labrafil^{®} M 1944 CS (cas #97488-91-0 et cas #9004-96-0) | 360 mg |
| Plurol^{®} oléique (cas #9007-48-1) | 4 g |
| Chlorhexidine base (cas #55-56-1) | 90 mg |
| Carboxyméthylcellulose sodique (cas #9004-32-4) | 1 g |
| Solution de Tween^{®} 80 à 0,15 % (cas #9005-65-5) | qsp 100 g |

Dans une première étape, le PCL est fondu à environ 65°C dans un bécher. Les huiles hydrodispersibles (Labrafil^{®} M 1944 CS et Plurol^{®} oléique) sont mélangées avec le PCL fondu, sous agitation mécanique modérée à l'aide d'un Ultra-Turrax^{®}. La chlorhexidine base est dispersée avec le PCL fondu et les huiles hydrodispersibles jusqu'à obtention d'un mélange limpide. Un hydrogel de carboxyméthylcellulose à 1% dans l'eau distillée est chauffé à 60°C afin d'être dispersé sous agitation mécanique modérée à l'aide d'un Ultra-Turrax^{®}, dans le mélange PCT fondu/huiles hydrodispersibles/chlorhexidine base. La formation des nanocapsules est spontanée sous l'effet de l'agrégation du PCL au contact de l'hydrogel. Cependant, la stabilité de la suspension de nanocapsules est maintenue par l'effet protecteur du colloïde hydrophile (i.e., carboxyméthylcellulose sodique) contre les phénomènes de floculation du PCL.

La taille et le potentiel zeta des nanocapsules, déterminés à l'aide d'un Zetamaster^{®} S, sont respectivement de 450 nm et de -40mV. La concentration de chlorhexidine libre (i.e., non encapsulée) dans la suspension de nanocapsules a été déterminée après filtration de la suspension colloïdale à travers un filtre 0,22 µm. Après dilution dans l'eau distillée, la concentration en chlorhexidine base libre dans le filtrat, déterminée par chromatographie liquide haute performance (CLHP) (H. Lboutounne, V. Faivre, F. Falson, and F. Pirot, Skin Pharmacology and Applied Skin Physiology 17: 176- 182 (2004)) ; H. Lboutounne, J.-F. Chaulet, C. Ploton, F. Falson, and F. Pirot Journal of Controlled Release 82: 319-334 (2002) ; D.T.T. Nhung, A.-M. Freydiere, H. Constant, F. Falson, and F. Pirot International Journal of Pharmaceutics 334:166-172 (2007)), était voisine de 0,008% soit la concentration à saturation de la chlorhexidine base dans l'eau. La concentration de chlorhexidine base encapsulée a été déterminée par différence entre la concentration totale de chlorhexidine base dans la suspension et la concentration de chlorhexidine libre. La concentration totale de chlorhexidine base encapsulée a été déterminée après dilution d'un aliquote de suspension colloïdale dans un mélange acétinitrile/acétate de sodium (30 mM) (50/50, v/v). La dilution dans ce mélange a pour effet de dissoudre les nanocapsules et de libérer la chlorhexidine base encapsulée. La concentration en chlorhexidine encapsulée était de 0,082% (pourcentage en masse/volume), soit un rendement d'encapsulation de près de 90%. L'aspect des nanocapsules de chlorhexidine base est présentée sur la **Figure unique** qui présente l'image obtenue par examen microscopique (x 1000) (Axioskop 50, Microscope Zeiss).

## Revendications

1. Procédé de préparation d'une suspension aqueuse de nanocapsules comprenant un coeur huileux entouré par une enveloppe polymérique dans lequel on mélange :
**a)** une première phase, nommée phase huileuse comportant :
• un polymère hydrophobe,
• une huile, ou un mélange d'huiles,
• au moins un principe actif,
• et un agent tensio-actif **TA₁**
cette phase huileuse étant portée à une température **T₁** supérieure à la température de fusion du polymère hydrophobe, à cette température **T₁,** le polymère hydrophobe étant miscible au mélange de l'agent tensio-actif **TA₁** et de l'huile ou du mélange d'huiles, et le principe actif étant miscible, soluble ou solubilisé, dans le mélange de l'agent tensio-actif **TA₁** et de l'huile ou du mélange d'huiles ;
**b)** une deuxième phase, nommée phase polaire, comportant un polymère hydrophile sous la forme d'un hydrogel dans une solution aqueuse contenant un agent tensio-actif **TA₂,**
de manière à obtenir la formation des nanocapsules en suspension aqueuse.

2. Procédé selon la revendication 1, **caractérisé en ce que** le mélange des deux phases est réalisé en ajoutant la phase polaire dans la phase huileuse, sous agitation.

3. Procédé selon la revendication 1 ou 2, **caractérisé en ce que** la phase polaire est portée à une température **T₂** inférieure de 2 à 5°C à la température de fusion du polymère hydrophobe.

4. Procédé selon l'une des revendications 1 à 3, **caractérisé en ce que** le principe actif est miscible, soluble ou solubilisé dans le mélange de l'agent tensio-actif **TA₁** et de l'huile ou du mélange d'huiles, à température ambiante, notamment à 20°C.

5. Procédé selon l'une des revendications 1 à 4, **caractérisé en ce que** l'huile ou le mélange d'huiles présente un HLB compris dans la gamme allant de 3 à 6.

6. Procédé selon l'une des revendications 1 à 5, **caractérisé en ce que** le principe actif est choisi parmi les actifs pharmaceutiques ou cosmétiques.

7. Procédé selon l'une des revendications 1 à 6, **caractérisé en ce que** la ou les huiles sont choisies, de préférence, parmi les triglycérides, notamment à chaîne moyenne, les propylène glycol dicaprylocaprates, les macrogolglycérides oléoyles, lauroyles et linoléoyles.

8. Procédé selon l'une des revendications 1 à 7, **caractérisé en ce que** le tensio-actif **TA₁** présente un HLB compris dans la gamme allant de 3 à 6, et est, de préférence, choisi parmi les propylène glycol laurates, les propylène glycol caprylates, les polyglycéryl oléates et les macrogolglycérides caprylocaproyles.

9. Procédé selon l'une des revendications 1 à 8, **caractérisé en ce que** le polymère hydrophobe est choisi parmi les polymères vinyliques, les polyesters, les polyamides, les polyuréthanes, les polycarbonates, présentant, de préférence, une température de fusion inférieure à 120°C, tels que les poly-ε-caprolactones.

10. Procédé selon l'une des revendications 1 à 9, **caractérisé en ce que** le polymère hydrophile est choisi parmi les dérivés cellulosiques synthétiques, de préférence parmi les éthers de cellulose tels que la méthylcellulose, l'éthylcellulose, l'hydroxyéthylcellulose, l'hydroxypropylcellulose, l'hydroxyéthyl-méthylcellulose, l'hydroxypropyl-méthylcellulose, la méthyléthylcellulose et la carboxyméthylcellulose sodique.

11. Procédé selon l'une des revendications 1 à 10, **caractérisé en ce que** le tensio-actif **TA₂** présente un HLB supérieur ou égal à 15, et est de préférence choisi parmi les tensio-actifs neutres.

12. Procédé selon l'une des revendications 1 à 11, **caractérisé en ce que** la phase huileuse comprend de 5 à 20% en masse, de préférence de 8 à 12 en masse de polymère hydrophobe.

13. Procédé selon l'une des revendications 1 à 12, **caractérisé en ce que** la phase huileuse comprend, de façon avantageuse, de 5 à 20% en masse, de préférence de 8 à 12% en masse d'huile ou mélange d'huiles.

14. Procédé selon l'une des revendications 1 à 13, **caractérisé en ce que** la phase huileuse comprend de 0,5 à 5% en masse, de préférence de 1 à 3% en masse de principe actif.

15. Procédé selon l'une des revendications 1 à 14, **caractérisé en ce que** la phase huileuse comprend de 55 à 89,5% en masse, de préférence de 73 à 83 % en masse de tensio-actif **TA₁**

16. Procédé selon l'une des revendications 1 à 15, **caractérisé en ce que** la phase polaire comprend de 0,1 à 5% en masse, de préférence de 0,15 à 2% en masse de polymère hydrophile.

17. Procédé selon l'une des revendications 1 à 16, **caractérisé en ce que** la phase polaire comprend de 90 à 99,8% en masse, de préférence de 96 à 99,65% en masse d'eau, ou d'un mélange eau/solvants polaires.

18. Procédé selon l'une des revendications 1 à 17, **caractérisé en ce que** la phase polaire comprend de 0,1 à 5% en masse, de préférence de 0,2 à 2% en masse de tensio-actif **TA₂.**

19. Procédé selon l'une des revendications 1 à 18, **caractérisé en ce que** le rapport massique polymère hydrophobe / polymère hydrophyle est inférieur ou égal à 0,4.

20. Procédé selon l'une des revendications 1 à 19, **caractérisé en ce que** les nanocapsules obtenues présentent un diamètre compris dans la gamme allant de 200 à 1000 nm, de préférence dans la gamme allant de 300 à 500 nm.

21. Nanocapsules comprenant un coeur huileux dans lequel un principe actif est dispersé de façon homogène et une enveloppe polymérique formée d'un polymère hydrophobe et d'un polymère hydrophile, susceptibles d'être obtenues selon le procédé tel que défini à l'une des revendications 1 à 20.

22. Nanocapsules selon la revendication 21, **caractérisée en ce que** le polymère hydrophile forme un colloïde protecteur venant enrober ou s'enchevêtrer avec le polymère hydrophobe formant l'enveloppe des nanocapsules.

23. Suspension aqueuse de nanocapsules selon l'une des revendications 21 ou 22.

24. Compositions pharmaceutique ou cosmétiques ou alimentaires, comprenant des nanocapsules selon la revendication 21 ou 22 ou une suspension aqueuse de nanocapsules selon la revendication 23, en combinaison avec au moins un excipient pharmaceutiquement ou physiologiquement acceptable.

## Patentansprüche

1. Verfahren zur Herstellung einer wässerigen Suspension von Nanokapseln, umfassend einen öligen Kern, der von einer Polymerhülle umgeben ist, bei dem gemischt werden:
a) eine erste Phase, ölige Phase genannt, umfassend:
* ein hydrophobes Polymer,
* ein Öl oder ein Ölgemisch,
* mindestens ein Aktivprinzip,
* und ein Tensid TA₁,
wobei diese ölige Phase auf eine Temperatur T₁ über der Schmelztemperatur des hydrophoben Polymer gebracht wird, wobei bei dieser Temperatur T₁ das hydrophobe Polymer mit dem Gemisch des Tensids TA₁ und des Öls oder des Ölgemisches mischbar ist, und wobei das Aktivprinzip in dem Gemisch des Tensids TA₁ und des Öls oder des Ölgemisches mischbar, löslich oder gelöst ist,
b) eine zweite Phase, polare Phase genannt, umfassend ein hydrophiles Polymer in Form eines Hydrogels in einer wässerigen Lösung, die ein Tensid TA₂ enthält,
um die Bildung von Nanokapseln in wässeriger Lösung zu erhalten.

2. Verfahren nach Anspruch 1, **dadurch gekennzeichnet, dass** das Gemisch der beiden Phasen hergestellt wird, wobei die polare Phase in die ölige Phase unter Schütteln hinzugefügt wird.

3. Verfahren nach Anspruch 1 oder 2, **dadurch gekennzeichnet, dass** die polare Phase auf eine Temperatur T₂ unter 2 bis 5°C bei der Schmelztemperatur des hydrophoben Polymers gebracht wird.

4. Verfahren nach einem der Ansprüche 1 bis 3, **dadurch gekennzeichnet, dass** das Aktivprinzip in dem Gemisch des Tensids TA₁ und des Öls oder des Ölgemisches bei Raumtemperatur, insbesondere bei 20°C, mischbar, löslich oder gelöst ist.

5. Verfahren nach einem der Ansprüche 1 bis 4, **dadurch gekennzeichnet, dass** das Öl oder das Ölgemisch einen HLB im Bereich von 3 bis 6 aufweist.

6. Verfahren nach einem der Ansprüche 1 bis 5, **dadurch gekennzeichnet, dass** das Aktivprinzip unter den pharmazeutischen oder kosmetischen Aktivstoffen ausgewählt ist.

7. Verfahren nach einem der Ansprüche 1 bis 6, **dadurch gekennzeichnet, dass** das oder die Öle vorzugsweise unter den Triglyceriden, insbesondere mittelkettig, den Propylenglykol-Dicaprylocapraten, den Oleoyl-, Lauroyl- und Linoleoyl-Makrogolglyceriden ausgewählt sind.

8. Verfahren nach einem der Ansprüche 1 bis 7, **dadurch gekennzeichnet, dass** das Tensid TA₁ einen HLB im Beriech von 3 bis 6 aufweist, und vorzugsweise unter den Propylenglykol-Lauraten, den Porpylenglykol-Caprylaten, den Polyglyceryl-Oleaten und den Caprylocaproyl- Makrogolglyceriden ausgewählt ist.

9. Verfahren nach einem der Ansprüche 1 bis 8, **dadurch gekennzeichnet, dass** das hydrophobe Polymer unter den Vinylpolymeren, den Polyestern, den Polyamiden, den Polyurethanen, den Polycarbonaten ausgewählt ist, die vorzugsweise eine Schmelztemperatur unter 120°C aufweisen, wie die Poly-έ-Caprolactone.

10. Verfahren nach einem der Ansprüche 1 bis 9, **dadurch gekennzeichnet, dass** das hydrophile Polymer unter den synthetischen Zellulosederivaten ausgewählt ist, vorzugsweise unter den Zelluloseethern, wie Methylzellulose, Ethylzellulose, Hydroxyethylzellulose, Hydroxypropylzellulose, Hydroxymethyl-Methylzellulose, Hydroxypropyl-Methylzelluose, Methylethylzellulose und Natrium-Carboxymethylzellulose.

11. Verfahren nach einem der Ansprüche 1 bis 10, **dadurch gekennzeichnet, dass** das Tensid TA₂ einen HLB größer oder gleich 15 aufweist und vorzugsweise unter den neutralen Tensiden ausgewählt ist.

12. Verfahren nach einem der Ansprüche 1 bis 11, **dadurch gekennzeichnet, dass** die ölige Phase 5 bis 20 Ma%, vorzugsweise 8 bis 12 Ma%, eines hydrophoben Polymers umfasst.

13. Verfahren nach einem der Ansprüche 1 bis 12, **dadurch gekennzeichnet, dass** die ölige Phase vorteilhafterweise 5 bis 20 Ma%, vorzugsweise 8 bis 12 Ma%, eines Öls oder Ölgemisches umfasst.

14. Verfahren nach einem der Ansprüche 1 bis 13, **dadurch gekennzeichnet, dass** die ölige Phase 0,5 bis 5 Ma%, vorzugsweise 1 bis 3 Ma%, eines Aktivprinzips umfasst.

15. Verfahren nach einem der Ansprüche 1 bis 14, **dadurch gekennzeichnet, dass** die ölige Phase 55 bis 89,5 Ma%, vorzugsweise 73 bis 83 Ma%, eines Tensids TA₁ umfasst.

16. Verfahren nach einem der Ansprüche 1 bis 15, **dadurch gekennzeichnet, dass** die polare Phase 0,1 bis 5 Ma%, vorzugsweise 0,15 bis 2 Ma%, eines hydrophilen Polymers umfasst.

17. Verfahren nach einem der Ansprüche 1 bis 16, **dadurch gekennzeichnet, dass** die polare Phase 90 bis 99,8 Ma%, vorzugsweise 96 bis 99,65 Ma%, Wasser oder eines Gemisches aus Wasser/polaren Lösungsmitteln umfasst.

18. Verfahren nach einem der Ansprüche 1 bis 17, **dadurch gekennzeichnet, dass** die polare Phase 0,1 bis 5 Ma%, vorzugsweise 0,2 bis 2 Ma% eines Tensids TA₂ umfasst.

19. Verfahren nach einem der Ansprüche 1 bis 18, **dadurch gekennzeichnet, dass** das Verhältnis hydrophobe Polymermasse / hydrophiles Polymer kleiner oder gleich 0,4 ist.

20. Verfahren nach einem der Ansprüche 1 bis 19, **dadurch gekennzeichnet, dass** die erhaltenen Nanokapseln einen Durchmesser aufweisen, der im Bereich von 200 bis 1000 nm, vorzugsweise im Bereich von 300 bis 500 nm, liegt.

21. Nanokapseln, umfassend einen öligen Kern, in dem ein Aktivprinzip homogen verteilt ist, und eine Polymerhülle, die von einem hydrophoben Polymer und einem hydrophilen Polymer gebildet ist, die nach dem Verfahren, wie in einem der Ansprüche 1 bis 20 definiert, erhalten werden können.

22. Nanokapseln nach Anspruch 21, **dadurch gekennzeichnet, dass** das hydrophile Polymer ein Schutzkolloid bildet, das das hydrophobe Polymer, das die Hülle der Nanokapseln bildet, umhüllt oder umschlingt.

23. Wässrige Lösung von Nanokapseln nach einem der Ansprüche 21 oder 22.

24. Pharmazeutische oder kosmetische Zusammensetzungen oder Lebensmittelzusammensetzungen, umfassend Nanokapseln nach Anspruch 21 oder 22 oder eine wässerige Lösung von Nanokapseln nach Anspruch 23, in Kombination mit mindestens einem pharmazeutisch oder physiologisch akzeptablen Exzipienten.

## Claims

1. A method for preparing an aqueous suspension of nanocapsules comprising an oily core surrounded by a polymeric shell, in which method the following phases are mixed:
**a)** a first phase, called an oily phase, comprising:
• a hydrophobic polymer,
• an oil or a mixture of oils,
• at least one active ingredient,
• and a surfactant **TA₁,**
this oily phase being brought to a temperature **T₁** higher than the melting point of the hydrophobic polymer, the hydrophobic polymer being miscible, at this temperature **T₁,** with the mixture of the surfactant **TA₁** and the oil or mixture of oils, and the active ingredient being miscible, soluble or solubilized in the mixture of the surfactant **TA₁** and the oil or mixture of oils;
**b)** a second phase, called a polar phase, comprising a hydrophilic polymer in the form of a hydrogel in an aqueous solution containing a surfactant **TA₂,**
in such a way as to obtain the formation of the nanocapsules in an aqueous suspension.

2. The method as claimed in claim 1, **characterized in that** the mixing of the two phases is carried out by adding the polar phase to the oily phase, with stirring.

3. The method as claimed in claim 1 or 2, **characterized in that** the polar phase is brought to a temperature **T₂** which is 2 to 5°C lower than the melting point of the hydrophobic polymer.

4. The method as claimed in one of claims 1 to 3, **characterized in that** the active ingredient is miscible, soluble or solubilized in the mixture of the surfactant **TA₁** and the oil or mixture of oils, at ambient temperature, in particular at 20°C.

5. The method as claimed in one of claims 1 to 4, **characterized in that** the oil or the mixture of oils has an HLB included in the range of from 3 to 6.

6. The method as claimed in one of claims 1 to 5, **characterized in that** the active ingredient is chosen from pharmaceutical or cosmetic active agents.

7. The method as claimed in one of claims 1 to 6, **characterized in that** the oil(s) is (are) preferably chosen from triglycerides, in particular medium-chain triglycerides, propylene glycol dicaprylocaprates, and oleoyl, lauroyl and linoleoyl macrogolglycerides.

8. The method as claimed in one of claims 1 to 7, **characterized in that** the surfactant **TA₁** has an HLB included in the range of from 3 to 6, and is preferably chosen from propylene glycol laurates, propylene glycol caprylates, polyglyceryl oleates and caprylocaproyl macrogolglycerides.

9. The method as claimed in one of claims 1 to 8, **characterized in that** the hydrophobic polymer is chosen from vinyl polymers, polyesters, polyamides, polyurethanes and polycarbonates, which preferably have a melting point lower than 120°C, such as poly-ε-caprolactones.

10. The method as claimed in one of claims 1 to 9, **characterized in that** the hydrophilic polymer is chosen from synthetic cellulosic derivatives, preferably from cellulose ethers, such as methylcellulose, ethylcellulose, hydroxyethylcellulose, hydroxypropylcellulose, hydroxyethylmethylcellulose, hydroxypropylmethylcellulose, methylethylcellulose and sodium carboxymethylcellulose.

11. The method as claimed in one of claims 1 to 10, **characterized in that** the surfactant **TA₂** has an HLB of greater than or equal to 15, and is preferably chosen from neutral surfactants.

12. The method as claimed in one of claims 1 to 11, **characterized in that** the oily phase comprises from 5% to 20% by mass, preferably from 8% to 12% by mass, of hydrophobic polymer.

13. The method as claimed in one of claims 1 to 12, **characterized in that** the oily phase advantageously comprises from 5% to 20% by mass, preferably from 8% to 12% by mass, of oil or mixture of oils.

14. The method as claimed in one of claims 1 to 13, **characterized in that** the oily phase comprises from 0.5% to 5% by mass, preferably from 1% to 3% by mass, of active ingredient.

15. The method as claimed in one of claims 1 to 14, **characterized in that** the oily phase comprises from 55% to 89.5% by mass, preferably from 73% to 83% by mass, of surfactant **TA₁.**

16. The method as claimed in one of claims 1 to 15, **characterized in that** the polar phase comprises from 0.1% to 5% by mass, preferably from 0.15% to 2% by mass, of hydrophilic polymer.

17. The method as claimed in one of claims 1 to 16, **characterized in that** the polar phase comprises from 90% to 99.8% by mass, preferably from 96% to 99.65% by mass, of water or of a water/polar solvents mixture.

18. The method as claimed in one of claims 1 to 17, **characterized in that** the polar phase comprises from 0.1% to 5% by mass, preferably from 0.2% to 2% by mass, of surfactant **TA₂**.

19. The method as claimed in one of claims 1 to 18, **characterized in that** the hydrophobic polymer/hydrophilic polymer mass ratio is less than or equal to 0.4.

20. The method as claimed in one of claims 1 to 19, **characterized in that** the nanocapsules obtained have a diameter included in the range of from 200 to 1000 nm, preferably in the range of from 300 to 500 nm.

21. Nanocapsules comprising an oily core in which an active ingredient is homogeneously dispersed and a polymeric shell formed from a hydrophobic polymer and from a hydrophilic polymer, which can be obtained according to the method as defined in one of claims 1 to 20.

22. The nanocapsules as claimed in claim 21, **characterized in that** the hydrophilic polymer forms a protective colloid which coats or is entangled with the hydrophobic polymer forming the shell of the nanocapsules.

23. An aqueous suspension of nanocapsules as claimed in either of claims 21 or 22.

24. Pharmaceutical or cosmetic or food compositions, comprising nanocapsules as claimed in claim 21 or 22 or an aqueous suspension of nanocapsules as claimed in claim 23, in combination with at least one pharmaceutically or physiologically acceptable excipient.
